# EUROPEAN PATENT SPECIFICATION

(11) **EP 1 949 894 B1**
(45) Date of publication and mention of the grant of the patent: **29.06.2016**
(21) Application number: 08001165.3
(22) Date of filing: 23.01.2008
(51) Int. Cl.: A61K 31/047, A61K 33/24, A61K 45/06, C12Q 1/42

(54) **Enhancement of vanadium-containing phosphatase inhibitors by polyols**
Verstärkung von vanadiumhaltigen Phosphatasehemmern mit Polyole
Amélioration des inhibiteurs de phosphatase contenant du vanadium avec des polyols

(30) Priority: 25.01.2007 EP 07001593
(43) Date of publication of application: 30.07.2008
(73) Proprietor: Roche Diagnostics GmbH, 68305 Mannheim (DE); F.Hoffmann-La Roche AG, 4070 Basel (CH)
(72) Inventor: Fernholz, Erhard, 82362 Weilheim (DE); Mayr, Dorothea, 82380 Peissenberg (DE)
(74) Representative: Merkel, Patrick

(56) References cited:
- EP-A- 0 051 964
- EP-A2- 0 524 633
- WO-A-00/57184
- WO-A-99/58097
- WO-A-03/029209
- WO-A-03/056029
- WO-A-2004/052412
- WO-A-2005/054257
- WO-A2-2007/075253
- US-A- 5 776 714
- US-A1- 2006 252 683
- US-B1- 6 299 865
- NAZ R ET AL: "An 18 kDa acid phosphatase from chicken heart possesses phosphotransferase activity", PROTEIN JOURNAL 2006 UNITED STATES, vol. 25, no. 2, 2006, pages 135-146, XP019401215,

## Description

The present invention relates to the biochemistry of phosphatases. Particularly, the invention deals with compounds which can be used as additives alongside vanadium-containing inhibitors of different phosphatase enzymes. The additive compounds according to the invention provide an enhancement of the inhibitory effect. As a consequence, lower concentrations of vanadium containing compounds can be used to achieve a sufficient inhibition of phosphatases.

### Background of the Invention

US 2006/0252683 A1 relates to the preparation of a peptide compound, its use to activate protein phosphatase-2A enzymes for the dephosphorylation of proteins in vitro and in the cell, and its use for inhibiting cell proliferation and induction of death of brain cancer cells and other cancer cells. In the document purification of protein phosphatase 2A from Jurkat cells is disclosed. The homogenization buffer contains sodium orthovanadate. WO 2003/029209 discloses pyrazolo-pyrimidine derivatives and uses thereof. In the document an assay using sodium orthovanadate as a tyrosine phosphatase inhibitor is disclosed. WO 1999/58097 relates to variant forms of human prolactin which act as antagonists at the prolactin receptor. The document discloses a cell lysis buffer containing sodium orthovanadate. WO 2000/57184 is directed to the characterization of interacting proteins originating from Saccharomyces cerevisiae. The document discloses a yeast protein extraction buffer with sodium orthovanadate. EP 0051964 discloses feedstuff additives which contain different vanadium compounds. WO 2004/052412 discloses wound dressings with organically complexed vanadate and peroxovanadate. WO 2005/054257 discloses vanadium compounds as phosphatase inhibitors, in combination with certain carrier substances. US 6299865 discloses reduction of mammalian hair growth by applying to the skin an inhibitor of alkaline phosphatase. In an embodiment sodium orthovanadate is used. EP 0524633 is directed to a pharmaceutical composition suitable for influencing the reticuloendothelic system and for treating mucoviscidosis and chronic pain syndromes deriving from degenerative locomotor diseases or accompanying the diseases of tumorous origin, the pharmaceutical composition containing vanadium in a pharmaceutically acceptable, water-soluble form.

US 5776714 discloses modified apo-horseradish peroxidase and, among other items, dry analytical elements having a porous spreading layer comprising vanadyl sulfate.

A phosphatase is an enzyme that hydrolyses phosphoric acid monoesters into a phosphate ion and a molecule with a free hydroxyl group. This action is directly opposite to that of phosphorylases and kinases, which attach phosphate groups to their substrates by using chemical energy conserving molecules like ATP.

Phosphatases can be categorised into two main categories: metalloenzymes (which are dependent on the presence of two or more metal ions in their active sites for activity), and non-metalloenzymes. These categories can then be divided into further sub-categories. The metalloenzymes by far comprise the greatest bulk of phosphatases, and contain such enzymes as alkaline phosphatase (three metal ions, only two of which are catalytically active), the serine threonine phosphatases, and inositol monophosphatase. Best known of the non-metalloenzymes are the protein tyrosine phosphatases, which hydrolyse phospho-tyrosine residues.

The presence or absence of a phosphate group on proteins is known to play a regulatory role in many biochemical and particularly signal transduction pathways. Tyrosine residues can be tagged with a phosphate group (phosphorylated) by protein kinases. In its phosphorylated state, the tyrosine residue is referred to as phosphotyrosine. Tyrosine phosphorylation is considered as one of the key steps in signal transduction and regulation of enzymatic activity. Phosphotyrosine can be detected through specific antibodies. Together, specialized kinases and phosphatases regulate the activity of enzymes, receptors and other components of signal transduction pathways, transcription factors, and other functional proteins.

Thus, there are numerous biochemical methods aimed at detecting phosphorylated proteins in a biological sample. For instance, sampled cell material is lysed and the protein fraction is subjected to one-dimensional (e.g. SDS-PAGE) or two-dimensional (e.g. 1^{st} dimension: isoelectric focussing, 2^{nd} dimension: SDS PAGE) separation and phosphorylated proteins of individual bands or spots are detected by phosphoserine- or phosphotyrosine-specific antibodies. Other more specific detection methods make use of antibodies which specifically bind to particular phosphorylated proteins. Such antibodies are frequently used for histocytochemical detection of phosphorylated target proteins in formalin fixed paraffin embedded tissue sections from biopsy material.

Regardless of the detection method used, the result of the analysis of phosphorylated proteins is desired to reflect the status of phosphorylation at the time point when the experiment is started, that is to say when the cells are lysed or the tissue is fixed and sectioned. More generally, it is desired to conserve the status of protein phosphorylation at a certain point in time. Conservation is achieved by preventing phosphate esters to be hydrolyzed from their target proteins. To this end, the state of the art provides a number of substances capable of inhibiting phosphatase activity. Inhibitors are applied routinely when performing assays for the detection of phosphorylated proteins but also when phosphorylated proteins are to be purified in larger amounts.

Among vanadium-containing inhibitors of phosphatase activity pervanadate and vanadate are the best known and most widely employed substances. Vanadate is a phosphate analogon which mimicks the transition state of phosphate hydrolysis and is therefore considered as a general phosphatase inhibitor. However, vanadate is recognized to be particularly suited to inhibit tyrosine phosphatases (Huyer, G., et al., J. Biol. Chem. 272 (1997) 843-851) and alkaline phosphatases (e.g. Stankiewicz, P. J., et al., in Met. Ions Biol. Syst. 31 (1995) 287-324). But inhibition of other phosphatase like ATPases, glucose-6 phosphatase, acid phosphatase or fructose-2,6-bisphosphatase had also been reported.

However, a disadvantage is that in order to provide an effective amount vanadate has to be in a concentration in the micromolar or even millimolar range. In this regard, an "effective amount" is understood as being between 1x (one time) and 50x (fifty times) the concentration of the inhibitor in an aqueous solution which at a 1x concentration reduces the activity of a phosphatase by the factor of 20. In contrast to vanadate, other inhibitors of phosphatase activity are known which are effective at nanomolar concentrations. An example therefor is Cantharidin.

It has been reported, however, that the inhibitory effect can be enhanced by way of forming stable vanadate-containing complexes. E.g. potassium bisperoxo (bipyridine) oxovanadate (V) and potassium bisperoxo (1,10 phenanthroline) oxovanadate (V)) both are more potent than orthovanadate. However, complexation of vanadate it not necessarily a prerequisite for improvement of potency. For instance, hydroxylamine or dimethylhydroxylamine spontaneously form complexes with vanadate. The potency of those complexes remain the same or is reduced to a certain extent (Cuncic, C., et al., Biochem. Pharmacol. 58 (1999) 1859-1867). In cell-based assays it was found that these two compounds increase the uptake of vanadate into the cellular lumen (Nxumalo, F., et al., J. Biol. Inorg. Chem. 3 (1998) 534-542; Cuncic, C., et al., Biochem. Pharmacol. 58 (1999) 1859-1867).

It is further known that in the presence of certain complex-forming reagents like EDTA the phosphatase inhibiting effect of vanadate is reduced by a factor of about 1,000 (Huyer, G., et al., J. Biol. Chem. 272 (1997) 843-851). This is particularly disadvantageous because EDTA is frequently used in biochemistry as a stabilizer and as an inhibitor of metalloenzymes such as phosphatases and proteases. Another import stabilizer for use in the preparation of cell lysates is dithiothreitol (DTT). The inventors have found that DTT also reduces to a significant extent the inhibitory effect of vanadate on phosphatases.

In view of the disadvantages of the state of the art it is an object of the present invention to provide alternative compounds which enhance the inhibitory effect of vanadate-containing compounds on enzymes with phosphatase activity. It is another object of the invention to provide compounds which counteract the negative effects of EDTA and DTT on vanadate-containing compounds.

The inventors have surprisingly found that in the presence of a polyol the inhibitory effect of vanadate on phosphatases and particularly on phosphotyrosine-specific phosphatases is potentiated. This effect was observed even with a very simple polyol like glycerol but also with sugar alcohols like mannitol. Even more surprising, the negative effects of EDTA and DTT on vanadate were found to be reduced significantly or even abolished completely.

### Summary of the Invention

A first embodiment of the invention is a liquid composition comprising an aqueous solvent, an enzyme with phosphatase activity, orthovanadate, mannitol and dithiothreitol, characterized in that the concentration of mannitol is between 1 mM and 100 mM, and the molar ratio of mannitol and orthovanadate is equal to or higher than 1:1. A second embodiment of the invention is an in vitro use of a water-soluble sugar alcohol with 4-100 carbon atoms for enhancing the inhibitory effect of an ionic compound selected from the group consisting of vanadate (V), oligomeric vanadate (V) and mixtures thereof on an enzyme with phosphatase activity capable of hydrolyzing the phosphoester bond of a phosphotyrosine residue in the peptide of SEQ ID NO:1 dissolved in a buffered aqueous solution with a neutral pH and containing Ca²⁺ ions at a concentration of between 0.7 mM and 1.2 mM. A third embodiment of the invention is a method in vitro to enhance the inhibitory effect of an ionic compound selected from the group consisting of vanadate (V), oligomeric vanadate (V) and mixtures thereof on an enzyme with phosphatase activity capable of hydrolyzing the phosphoester bond of a phosphotyrosine residue in the peptide of SEQ ID NO:1 dissolved in a buffered aqueous solution with a neutral pH and containing Ca²⁺ ions at a concentration of between 0.7 mM and 1.2 mM, comprising the steps of (a) dissolving in an aqueous solvent a composition comprising (i) the ionic compound, and (ii) a water-soluble sugar alcohol with 4-100 carbon atoms, whereby the molar ratio of the sugar alcohol and the ionic compound is equal to or higher than 1:1, and (b) contacting the enzyme with phosphatase activity with the solution of step (a), A fourth embodiment of the invention is a kit of parts comprising packaging material and a composition comprising (i) an ionic compound selected from the group consisting of vanadate (V), oligomeric vanadate (V) and mixtures thereof, (ii) a water-soluble sugar alcohol with 4-100 carbon atoms, whereby the molar ratio of the sugar alcohol and the ionic compound is equal to or higher than 1:1.

### Detailed Description of the Invention

Certain terms are used with particular meaning, or are defined for the first time, in this description of the present invention. For the purposes of understanding the present invention, the terms used to describe the invention are defined by their art-accepted definitions, when such exist, except that when those definitions conflict or partially conflict with the definitions set forth below. In the event of a conflict in definition, the meaning of the terms are first defined by the definitions set forth below.

The term "comprising" is used in the description of the invention and in the claims to mean "including, but not necessarily limited to". The indefinite article "a" in combination with a term denoting a chemical compound such as an inhibitor or an additive is used to denote "one or more". A "phosphatase inhibitor" is a substance which is effective to inhibit the hydrolysis of phosphoesters by phosphatase enzymatic activity, and thereby the release of phosphate ions from a target molecule. An "aqueous" solution is understood as a solution wherein the liquid solvent is an aqueous solvent which comprises at least 80% [v/v] water, more preferred 95% [v/v], even more preferred 99% [v/v], yet even more preferred 100% [v/v]. The skilled person appreciates that the solution further comprises one or more further compounds such as a salt, a buffer, an inhibitor, an additive, and a biological molecule, whereby the one or more compounds are dissolved in the liquid solvent.

A composition according to the present disclosure comprises an ionic compound selected from the group consisting of vanadyl (II), vanadate (IV), vanadate (V), oligomeric vanadate (V), and mixtures thereof. Very much preferred, the ionic vanadium-containing compound is orthovanadate (V) and oligomers thereof. A preferred oligomer is selected from the group consisting of a di-, tri-, and tetravananadate ion. Another very much preferred ionic compound in the composition according to the invention is a peroxovanadate ion. However, most preferred is orthovanadate (VO₄³⁻).

The "polyol" in the composition according to the invention is a water-soluble organic compound in which two or more hydroxyl groups are covalently linked with carbon atoms. It is preferred that two of the hydroxyl groups of the polyol according to the invention are bonded to two adjacent carbon atoms. In other words, the preferred polyol comprises two vicinal hydroxyl groups. However, polyols with more than two vicinal hydroxyl groups are preferred. Very well known polyols according to claimed invention with vicinal hydroxyl groups are sugar alcohols. A "sugar alcohol" is a hydrogenated form of carbohydrate, whose carbonyl group (aldehyde or ketone) has been reduced to a primary or secondary hydroxyl group.

The unhydrogenated form of the carbohydrate is also referred to as a "reducing" sugar.

The composition according to the invention comprises a sugar alcohol with 4-100 carbon atoms. In this respect, non-reducing mono-, di-, tri- and tetrasaccharides are very much preferred.

A very much preferred sugar alcohol is a non-reducing monosaccharide. Such a sugar alcohol can be a C4 sugar alcohol and preferably selected from the group consisting of threitol and eythritol. Also very much preferred, the sugar alcohol is a C5 sugar alcohols and preferably selected from the group consisting of ribitol, arabitol, xylitol, and lyxitol. Also very much preferred, the sugar alcohol is a C5 deoxy sugar alcohol and preferably selected from the group consisting of deoxyribitol and dexoyarabitol. Also very much preferred, the sugar alcohol is a C6 sugar alcohol and preferably selected from the group consisting of allitol, altritol, mannitol, glucitol, glutol, iditol, galactitol, and talitol.

According to the invention, a deoxy sugar alcohol is encompassed by the term sugar alcohol provided that the deoxy sugar provides four or more pairs of vicinal hydroxyl groups. A very much preferred sugar alcohol is a C6 deoxy sugar alcohol and preferably selected from the group consisting of deoxyglucitol, and deoxymannitol. Also very much preferred, the sugar alcohol is a sugar alcohol with other substituents in the C2-position of which a preferred example is N-acetyl glucitol amine 2. with other substituents on C2-position like N-acetyl glucitol amine-2.

Disaccharides and monosaccharides can both form sugar alcohols; however, sugar alcohols derived from disaccharides (e.g. maltitol and lactitol) are not entirely hydrogenated because due to the glycosidic bond only one aldehyde group is available for reduction. The same applies to trisaccharides and higher saccharides. Thus, a non-reducible di- or trisaccharide, or a non-reducing higher oligosaccharide with up to 100 C atoms can be used with great advantage to practice the invention. Sucrose is a highly preferred sugar alcohol. What is noteworthy about sucrose is that unlike most polysaccharides, the glycosidic bond is formed between the reducing ends of both glucose and fructose, and not between the reducing end of one and the nonreducing end of the other. The effect of this inhibits further bonding to other saccharide units. Since it contains no free anomeric carbon atom, it is a nonreducing sugar.

According to the invention, the preferred molar ratio between the polyol and the ionic vanadium-containing compound is higher than 1:1. Very much preferred, the molar ratio is between 100:1 and 1:1. Even more preferred, the molar ratio is between 70:1 and 5:1. Even more preferred, the molar ratio is between 50:1 and 10:1.

Taking phosphatase activity in a sample in the absence of a vanadium containing inhibitor as a reference (100%) the combination of an ionic compound selected from the group consisting of vanadyl (II), vanadate (IV), vanadate (V), oligomeric vanadate (V) and a polyol according to the invention, when added to the sample, is capable to substantially reduce said phosphatase activity. Preferably, said activity is reduced to a value between 1.5% and 40%, more preferred to a value of between 1.5% and 30%, even more preferred to a value of between 1.5% and 20%, even more preferred to a value of between 1.5% and 10%.

On the one hand, the polyol enhances the inhibitory effect of the ionic vanadium-containing compound on phosphatase activity. On the other hand, the polyol reduces the negative effect of complex-forming agents. That is to say inhibition of phosphatase activity is not adversely affected by the presence of a chelating agent for divalent or trivalent positively charged metal ions. Complex formation of divalent ions may therefore e.g. prevent undesired activity of a number of proteases without side effects. For this reason, the composition of the invention additionally comprises a chelating agent for divalent or trivalent positively charged metal ions. A preferred chelating agent is selected from the group consisting of EDTA, citrate, EGTA, and 1,10-phenanthroline. The preferred concentration of the chelating agent in the composition according to the invention is between 0.1 mM and 50 mM, more preferred between 0.2 mM and 10 mM, and most preferred at about 1 mM.

Furthermore, the polyol in the composition reduces the negative effect of reducing agents on the ionic vanadium-containing compound. Particularly DTT reduces the inhibition of phosphatase activity by orthovanadate. However, in the presence of a polyol the reduction is reversed. A preferred reducing agent is selected from the group consisting of DTT (dithiothreitol), beta-mercaptoethanol, glutathione, and thioredoxine. The preferred concentration of the reducing agent in the composition according to the invention is between 0.1 mM and 30 mM, more preferred between 0.2 mM and 10 mM, and most preferred at about 1 mM.

In another embodiment of the invention, the composition is provided for the end-user in a convenient form. An example therefor is a package containing a measured quantity of the composition. The packaging material is preferably selected to prevent contact with water of water vapour. In addition, one or more packages can be stored in the presence of drying material such as silica gel or other suitable substances. The composition can be in the form of a free-flowing granulate. Even more preferred, the composition is in the form of a tablet. In this case, the composition may in addition contain further materials which facilitate tablet formation.

In addition, the composition of the invention may contain one or more additional phosphatase inhibitor which is a compound other than a vanadium-containing compound. The one or more additional phosphatase inhibitor is preferably an ionic inhibitor like NaF. Also preferred, the inhibitor is a low molecular weight compound selected from the group consisting of canthridin, napthyl phosphate, and microcystin. Also preferred, the inhibitor is a peptidic compound selected from the group consisting of calcineurin autoinhibitory peptide, and protein phosphatase inhibitor 2.

Owing to the surprising effect of the presence of a polyol in addition to the vanadate-containing compound, the composition according to the invention is suitable for the inhibition of an enzyme with phosphatase activity. The composition is particularly suited to inhibit a phosphatase selected from the group consisting of an acid phosphatase, an alkaline phosphatase, a phosphotyrosine phosphatase, an ATPase, a phosphoserin/threonin phosphatase, and a dual phosphatase. Most preferred, the composition is used to inhibit a protein tyrosine phosphatase, that is to say to hydrolyze the phosphate ester of a phosphotyrosine resudue, whereby the phosphotyrosine residue is part of a phosphorylated polypeptide. Examples for polypeptides which may contain a phosphorylated tyrosine residue and which are a substrate for a phosphotyrosine-specific protein phosphatase are the phosphorylated receptor of erythropoietin, pErk1, pErk2, and pjak2.

Another embodiment of the disclosure herein is thus a method to inhibit an enzyme with phosphatase activity, comprising the steps of (a) dissolving in an aqueous solvent a composition comprising (i) an ionic compound selected from the group consisting of vanadyl (II), vanadate (IV), vanadate (V), oligomeric vanadate (V), and mixtures thereof, and (ii) a polyol, and (b) contacting the enzyme with phosphatase activity with the solution of step (a).

A further embodiment of the disclosure herein is therefore a liquid composition comprising the composition according to the invention and an aqueous solvent. The pH of the liquid composition preferably corresponds to a pH value at which a phosphatase enzyme (one or more target phosphatases) is active. The pH is preferably in the range of pH 5.5 - pH 8.5, more preferred in the range of pH 6.7 - pH 8.0.

The following examples, figures and sequence listing are provided to aid the understanding of the present invention, the true scope of which is set forth in the appended claims.

### Description of the Figures

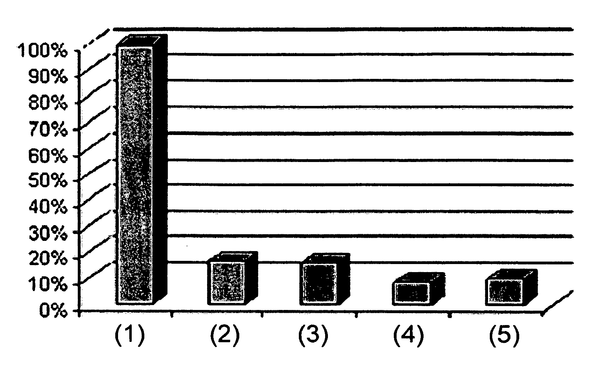

- **Figure 1**: Inhibition of a phosphotyrosine-specific protein phosphatase with orthovanadate in the presence or absence of a polyol. For the principle of the experiment reference is made to Example 1. The ordinate indicates residual phosphatase activity. The bars indicate the results obtained for the following compositions: (1) no inhibitor added (control, 100% residual activity); (2) 1 mM orthovanadate; (3) 2 mM orthovanadate; (4) 1 mM orthovanadate, 27 mM mannitol; (5) 1 mM orthovanadate, 54 mM glycerol.

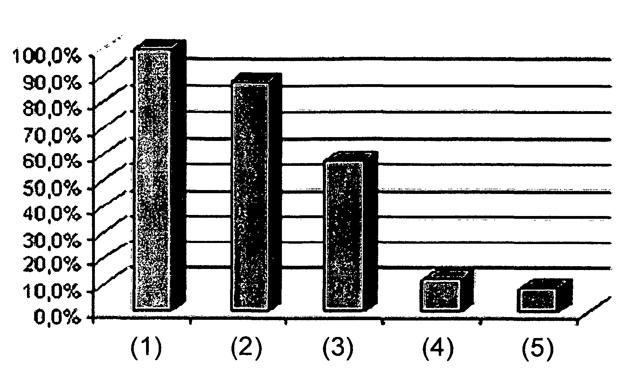

- **Figure 2**: Inhibition of a phosphotyrosine-specific protein phosphatase with orthovanadate in the presence or absence of a polyol and in the presence of EDTA and DTT. For the principle of the experiment reference is made to Example 2. The ordinate indicates residual phosphatase activity. The bars indicate the results obtained for the following compositions: (1) no inhibitor added (control, 100% residual activity); (2) 1 mM orthovanadate; (3) 2 mM orthovanadate; (4) 1 mM orthovanadate, 27 mM mannitol; (5) 1 mM orthovanadate, 54 mM glycerol.

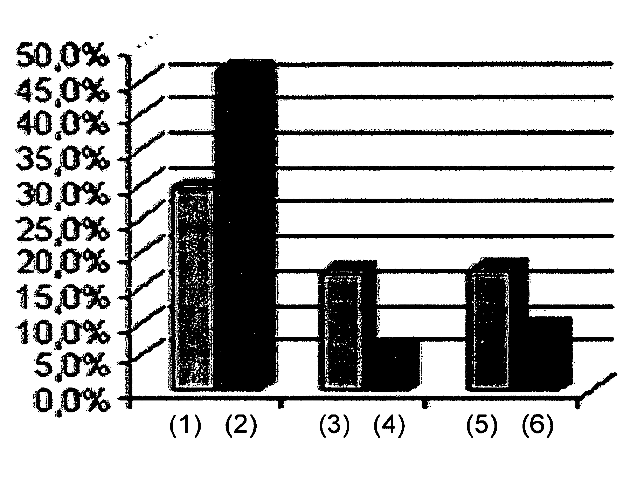

- **Figure 3**: Inhibition of a phosphotyrosine-specific protein phosphatase with orthovanadate in the presence or absence of a polyol and in the presence of EDTA, with or without DTT. For the principle of the experiment reference is made to Example 3. The ordinate indicates residual phosphatase activity. The bars indicate the results obtained for the following compositions: (1) 1 mM orthovanadate, Hepes buffer with EDTA; (2) 1 mM orthovanadate, Hepes buffer with EDTA and DTT; (3) 1 mM orthovanadate, Hepes buffer with EDTA, in the presence of 27 mM mannitol; (4) 1 mM orthovanadate, Hepes buffer with EDTA and DTT, in the presence of 27 mM mannitol; (5) 1 mM orthovanadate, Hepes buffer with EDTA, in the presence of 54 mM glycerol; (6) 1 mM orthovanadate, Hepes buffer with EDTA and DTT, in the presence of 54 mM glycerol.

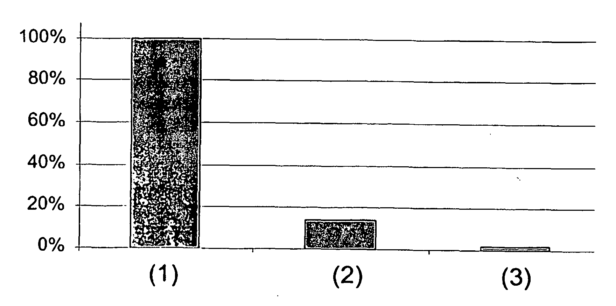

- **Figure 4**: Inhibition of phosphatise activity in insect cell lysate with orthovanadate in the presence or absence of mannitol. For the principle of the experiment reference is made to Example 5. The ordinate indicates residual phosphatase activity. The bars indicate the results obtained for the following compositions: (1) no inhibitor added (control, 100% residual activity); (2) 1 mM orthovanadate; (3) 1 mM orthovanadate, 27 mM mannitol.

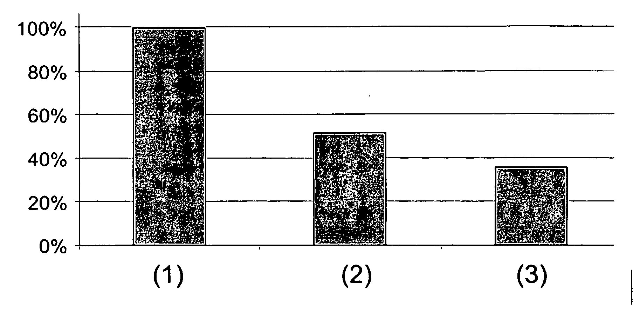

- **Figure 5**: Inhibition of phosphatise activity in COS7 cell lysate with orthovanadate in the presence or absence of mannitol. For the principle of the experiment reference is made to Example 6. The ordinate indicates residual phosphatase activity. The bars indicate the results obtained for the following compositions: (1) no inhibitor added (control, 100% residual activity); (2) 1 mM orthovanadate; (3) 1 mM orthovanadate, 27 mM mannitol.

### Example 1

### Inhibition of a phosphotyrosine-specific protein phosphatase

A stock solution of a recombinantly produced phosphotyrosine-specific protein phosphatase (human T-cell; Calbiochem , No. Cat 539732) is diluted in the ratio of 1:200 in a buffer containing 0,1 mM CaCl₂, 50 mM Tris pH 7.0. In separate reaction tubes the following mixtures (a-e) are prepared: 36 µl of diluted enzyme solution and 4 µl of either of (a) water (as a reference for 100%) one solution of (b) 10 mM orthovanadate; (c) 20 mM orthovanadate; (d) 10 mM orthovanadate, 270 mM mannitol; (e) 10 mM orthovanadate, 540 mM glycerol. Each mixture is incubated at room temperature (RT) for 15 min. Subsequently, an aliquot of 10 µl is transferred from each mixture to a microwell plate. 5 µl of a solution of a test peptide containing a phosphotyrosine residue (the peptide having the sequence R-R-L-I-E-D-A-E-pY-A-A-R-G; 1 mM in water) and 10 µl of a buffer containing 0,1 mM CaCl₂, 50 mM Tris pH 7.0 are added to each well and incubated for 30 min at 37°C. Free phosphate resulting from enzymatic hydrolysis of the phosphoester bond is subjected to a detection reaction following the addition of 100 µl of a 1 M HCl solution additionally containing 0.034% [w/v] malachite green, 10 mM sodium molybdate, and 3.4% [v/v] ethanol. The detection reaction is performed for 10 min at RT while constantly agitating the microwell plate. Relative concentrations of phosphate are detected following the determination of the extinction (620 nm) of each reaction well.

### Example 2

### Inhibition of a phosphotyrosine-specific protein phosphatase in the presence of EDTA and DTT

A stock solution of a recombinantly produced phosphotyrosine-specific protein phosphatase (human T-cell; Calbiochem , No. Cat 539732) is diluted in the ratio of 1:200 in a buffer containing 25 mM Hepes, 50 mM NaCl, 2.5 mM EDTA, 5 mM DTT, pH 7.2. In separate reaction tubes the following mixtures (a-e) are prepared: 36 µl of diluted enzyme solution and 4 µl of either of (a) water (as a reference for 100%) one solution of (b) 10 mM orthovanadate; (c) 20 mM orthovanadate; (d) 10 mM orthovanadate, 270 mM mannitol; (e) 10 mM orthovanadate, 540 mM glycerol. Each mixture is incubated at room temperature (RT) for 15 min. Subsequently, an aliquot of 10 µl is transferred from each mixture to a microwell plate. 5 µl of a solution of a test peptide containing a phosphotyrosine residue (the peptide having the sequence R-R-L-I-E-D-A-E-pY-A-A-R-G; 1 mM in water) and 10 µl of a buffer containing 25 mM Hepes, 50 mM NaCl, 2.5 mM EDTA, 5 mM DTT, pH 7.2 are added to each well and incubated for 30 min at 37°C. Free phosphate resulting from enzymatic hydrolysis of the phosphoester bond is subjected to a detection reaction following the addition of 100 µl of a 1 M HCl solution additionally containing 0.034% [w/v] malachite green, 10 mM sodium molybdate, and 3.4% [v/v] ethanol. The detection reaction is performed for 10 min at RT while constantly agitating the microwell plate. Relative concentrations of phosphate are detected following the determination of the extinction (620 nm) of each reaction well.

### Example 3

### Inhibition of a phosphotyrosine-specific protein phosphatase in the presence of EDTA, whereby DTT is either present additionally or absent

A stock solution of a recombinantly produced phosphotyrosine-specific protein phosphatase (human T-cell; Calbiochem , No. Cat 539732) is diluted in the ratio of 1:200, either in a buffer containing 25 mM Hepes, 50 mM NaCl, 2.5 mM EDTA, 5 mM DTT, pH 7.2 (enzyme solution (i)) or in a buffer with the same composition but without DTT (enzyme solution (ii)). In separate reaction tubes the following mixtures (a-f) are prepared: 36 µl of either diluted enzyme solution (i) or (ii) and 4 µl of either one solution of (a, d) 10 mM orthovanadate; (b, e) 10 mM orthovanadate, 270 mM mannitol; (c, f) 10 mM orthovanadate, 540 mM glycerol. Each mixture is incubated at room temperature (RT) for 15 min. Subsequently, an aliquot of 10 µl is transferred from each mixture to a microwell plate. 5 µl of a solution of a test peptide containing a phosphotyrosine residue (the peptide having the sequence R-R-L-I-E-D-A-E-pY-A-A-R-G; 1 mM in water) and either 10 µl of a buffer containing 25 mM Hepes, 50 mM NaCl, 2.5 mM EDTA, 5 mM DTT, pH 7.2 (reactions with enzyme solution (i)) or 10 µl of a buffer containing 25 mM Hepes, 50 mM NaCl, 2.5 mM EDTA, pH 7.2 (reactions with enzyme solution (ii)) are added to each well and incubated for 30 min at 37°C. Free phosphate resulting from enzymatic hydrolysis of the phosphoester bond is subjected to a detection reaction following the addition of 100 µl of a 1 M HCl solution additionally containing 0.034% [w/v] malachite green, 10 mM sodium molybdate, and 3.4% [v/v] ethanol. The detection reaction is performed for 10 min at RT while constantly agitating the microwell plate. Relative concentrations of phosphate are detected following the determination of the extinction (620 nm) of each reaction well.

### Example 4

### Inhibition of a Phosphotyrosine-specific protein phosphatase with orthovanadate

**Table 1**

| Residual phosphatase activity in the presence of compositions containing orthovanadate and other compounds in aqueous buffers as indicated. The tabulated data refer to the graphic representations given in Figures 1-3. | | |
|---|---|---|
| | **Buffer** | **Activity** |
| Figure 1: | | |
| 1) Without Inhibitor | 50 mM Tris; 0,1 mM CaCl₂ pH 7,0 | 100% |
| 2) Na₃VO₄ 1 mM | 50 mM Tris; 0,1 mM CaCl₂ pH 7,0 | 16,6% |
| 3) Na₃VO₄ 2 mM | 50 mM Tris; 0,1 mM CaCl₂ pH 7,0 | 15,7% |
| 4) Na₃VO₄ 1 mM + mannitol 27 mM | 50 mM Tris; 0,1 mM CaCl₂ pH 7,0 | 8,5% |
| 5) Na₃VO₄ 1 mM + glycerol 54 mM | 50 mM Tris; 0,1 mM CaCl₂ pH 7,0 | 9,7% |

| Figure 2: | | |
|---|---|---|
| 1) Without Inhibitor | 25 mM Hepes ; 50 mM NaCl; 2,5 mM EDTA ; 5 mM DTT pH 7,2 | 100% |
| 2) Na₃VO₄ 1 mM | 25 mM Hepes ; 50 mM NaCl; 2,5 mM EDTA; 5 mM DTT pH 7,2 | 87,7% |
| 3) Na₃VO₄ 2 mM | 25 mM Hepes ; 50 mM NaCl; 2,5 mM EDTA ; 5 mM DTT pH 7,2 | 58,0% |
| 4) Na₃VO₄ 1 mM + mannitol 27 mM | 25 mM Hepes ; 50 mM NaCl; 2,5 mM EDTA; 5 mM DTT pH 7,2 | 12,4% |
| 5) Na₃VO₄ 1 mM + glycerol 54 mM | 25 mM Hepes ; 50 mM NaCl; 2,5 mM EDTA ; 5 mM DTT pH 7,2 | 9,3% |

| **Figure 3****:** | | |
|---|---|---|
| 1) Na₃VO₄ 1 mM | 25 mM Hepes ; 50 mM NaCl; 2,5 mM EDTA; pH 7,2 | 29,5% |
| 2) Na₃VO₄ 1 mM | 25 mM Hepes ; 50 mM NaCl; 2,5 mM EDTA; S mM DTT pH 7,2 | 46,3% |
| 3) Na₃VO₄ 1 mM + mannitol 27 mM | 25 mM Hepes ; 50 mM NaCl; 2,5 mM EDTA ; pH 7,2 | 14,1% |
| 4) Na₃VO₄ 1 mM + mannitol 27 mM | 25 mM Hepes ; 50 mM NaCl; 2,5 mM EDTA; 5 mM DTT pH 7,2 | 5,8% |
| 5) Na₃VO₄ 1 mM + glycerol 54 mM | 25 mM Hepes ; 50 mM NaCl; 2,5 mM EDTA ; pH 7,2 | 14,9% |
| 6) Na₃VO₄ 1 mM + glycerol 54 mM | 25 mM Hepes ; 50 mM NaCl; 2,5 mM EDTA ; 5 mM DTT pH 7,2 | 7,3% |

### Example 5

### Inhibition of phosphatases in insect cell lysate

270 mg insect cells (SF9) were collected, washed 3 times with 50mM Hepes, 50mM NaCl pH 7.0 and lysed using 2.5 ml M-Per (Pierce) for 10 min. After centrifugation the lysate was collected as the supernatant.

Test for phosphotyrosine activity: to 45µl of the lysate 5µl of either water or orthovanadate (10 mM) or orthovanadate/mannitol (10 mM/270 mM) were added and incubated for 10 min at room temperature.

Subsequently, an aliquot of 10 µl was transferred from each mixture into a well of a microwell plate. 5µl of a solution of a test peptide (R-R-L-I-E-D-A-E-pY-A-A-R-G; 1mM in water) and 10 µl of a buffer containing 0.1 mM CaCl₂, 50 mM Tris pH 7.0 were added to each well and incubated for 30 min at 37°C. Free phosphate resulting from the enzymatic hydrolysis was then detected via addition of 100 µl of a 1 M HCl solution additionally containing 0.034% [w/v] malachite green, 10 mM sodium molybdate and 3.4% [v/v] ethanol. The detection was performed for 10 min at RT while constantly agitating the microwell plate. Relative concentrations of free phosphate were detected following the determination of the extinction (620 nm) of each reaction well.

**Table 2**

| Residual phosphatase activity in the presence of compositions containing orthovanadate and other compounds in aqueous buffers as indicated. The tabulated data refer to the graphic representations given in Figure 4. | |
|---|---|
| | **phosphatase activity** |
| no inhibitor (water control) | 100% |
| 1 mM orthovandate | 14% |
| 1 mM orthovanadate / 27 mM mannitol | 2% |

### Example 6

### Inhibition of phosphatases in COS7 cell lysate

COS7 cells were collected, washed 3 times with 50mM Hepes, 50mM NaCl pH 7.0 and lysed using 5 ml M-Per (Pierce) for 10 min. After centrifugation the lysate was collected as the supernatant.

Test for phosphotyrosine activity: to 45 µl of the diluted lysate (1+4 with M-Per (Pierce)) 5 µl of either water or orthovanadate (10 mM) or orthovanadate/Mannitol (10 mM/270 mM) were added and incubated for 10 min at room temperature.

Subsequently, an aliquot of 10µl was transferred from each mixture to a microwell plate. 5 µl of a solution of a test peptide (R-R-L-I-E-D-A-E-pY-A-A-R-G; 1 mM in water) and 10 µl of a buffer containing 0.1 mM CaCl₂, 50mM Tris pH 7.0 were added to each well and incubated for 30 min at 37°C. Free phosphate resulting from the enzymatic hydrolysis was then detected via addition of 100 µl of a 1M HCl solution additionally containing 0.034% [w/v] malachite green, 10 mM sodium molybdate and 3.4 [v/v] ethanol. The detection was performed for 10 min at RT while constantly agitating the microwell plate. Relative concentrations of free phosphate were detected following the determination of the extinction (620nm) of each reaction well.

**Table 3**

| Residual phosphatase activity in the presence of compositions containing orthovanadate and other compounds in aqueous buffers as indicated. The tabulated data refer to the graphic representations given in Figure 5. | |
|---|---|
| | **phosphatase activity** |
| no inhibitor (water control) | 100% |
| 1 mM orthovandate | 52% |
| 1 mM orthovanadate / 27 mM mannitol | 36% |

## Claims

1. A liquid composition comprising an aqueous solvent, an enzyme with phosphatase activity, orthovanadate, mannitol and dithiothreitol, **characterized in that** the concentration of mannitol is between 1 mM and 100 mM, and the molar ratio of mannitol and orthovanadate is equal to or higher than 1:1.

2. The composition according to claim 1, **characterized in that** the concentration of orthovanadate is between 0.1 mM and 10 mM.

3. The composition according to claim 2, **characterized in that** the concentration of orthovanadate is between 0.5 mM and 5 mM.

4. The composition according to any of the claims 1 to 3, **characterized in that** the composition additionally comprises a chelating agent for divalent or trivalent positively charged metal ions.

5. In vitro use of a water-soluble sugar alcohol with 4-100 carbon atoms selected from the group consisting of threitol, erythritol, ribitol, arabitol, xylitol, lyxitol, deoxyribitol, deoxyarabitol, allitol, altritol, mannitol, glucitol, gulitol, iditol, galactitol, talitol, deoxyglucitol, deoxymannitol, N-acetyl glucitol-amine-2, maltitol, and lactitol for enhancing the inhibitory effect of an ionic compound selected from the group consisting of vanadate (V), oligomeric vanadate (V) and mixtures thereof on an enzyme with phosphatase activity capable of hydrolyzing the phosphoester bond of a phosphotyrosine residue in the peptide of SEQ ID NO:1 dissolved in a buffered aqueous solution with a neutral pH and containing Ca²⁺ ions at a concentration of between 0.7 mM and 1.2 mM.

6. The use according to claim 5, **characterized in that** the activity of the phosphatase is reduced to a value of between 1.5% and 40%.

7. A method in vitro to enhance the inhibitory effect of an ionic compound selected from the group consisting of vanadate (V), oligomeric vanadate (V) and mixtures thereof on an enzyme with phosphatase activity capable of hydrolyzing the phosphoester bond of a phosphotyrosine residue in the peptide of SEQ ID NO:1 dissolved in a buffered aqueous solution with a neutral pH and containing Ca²⁺ ions at a concentration of between 0.7 mM and 1.2 mM, comprising the steps of
(a) dissolving in an aqueous solvent a composition comprising (i) the ionic compound, and (ii) a water-soluble sugar alcohol with 4-100 carbon atoms selected from the group consisting of threitol, erythritol, ribitol, arabitol, xylitol, lyxitol, deoxyribitol, deoxyarabitol, allitol, altritol, mannitol, glucitol, gulitol, iditol, galactitol, talitol, deoxyglucitol, deoxymannitol, N-acetyl glucitol-amine-2, maltitol, and lactitol, whereby the molar ratio of the sugar alcohol and the ionic compound is equal to or higher than 1:1, and
(b) contacting the enzyme with phosphatase activity with the solution of step (a).

8. The method according to claim 7, **characterized in that** the concentration of the sugar alcohol in the solution is between 1 mM and 100 mM.

9. The method according to any of claims 7 and 8, **characterized in that** the concentration of orthovanadate in the solution is between 0.1 mM and 10 mM.

10. The method according to any of claims 7 to 9, **characterized in that** the ionic compound and the sugar alcohol are added as dry matter to an aqueous sample containing the enzyme with phosphatase activity.

11. A kit of parts comprising packaging material and a composition comprising (i) an ionic compound selected from the group consisting of vanadate (V), oligomeric vanadate (V) and mixtures thereof, (ii) a water-soluble sugar alcohol with 4-100 carbon atoms, whereby the molar ratio of the sugar alcohol and the ionic compound is equal to or higher than 1:1.

12. The kit according to claim 11, **characterized in that** the composition additionally comprises a reducing agent and/or a chelating agent for divalent or trivalent positively charged metal ions.

13. The Kit according to any of the claims 11 and 12, **characterized in that** the composition is provided as dry matter in the form of a tablet.

## Patentansprüche

1. Flüssige Zusammensetzung, umfassend ein wässriges Lösungsmittel, ein Enzym mit Phosphatase-Aktivität, Orthovanadat, Mannitol und Dithiothreitol, **dadurch gekennzeichnet, dass** die Mannitol-Konzentration zwischen 1 mM und 100 mM liegt und das Molverhältnis von Mannitol und Orthovanadat gleich oder höher als 1:1 ist.

2. Zusammensetzung nach Anspruch 1, **dadurch gekennzeichnet, dass** die Orthovanadat-Konzentration zwischen 0,1 mM und 10 mM liegt.

3. Zusammensetzung nach Anspruch 2, **dadurch gekennzeichnet, dass** die Orthovanadat-Konzentration zwischen 0,5 mM und 5 mM liegt.

4. Zusammensetzung nach einem der Ansprüche 1 bis 3, **dadurch gekennzeichnet, dass** die Zusammensetzung zusätzlich einen Chelatbildner für zweiwertige oder dreiwertige positiv geladene Metallionen umfasst.

5. In-vitro-Verwendung eines wasserlöslichen Zuckeralkohols mit 4-100 Kohlenstoffatomen, ausgewählt aus der Gruppe bestehend aus Threitol, Eythritol, Ribitol, Arabinitol, Xylitol, Lyxitol, Desoxyribitol, Desoxyarabinitol, Allitol, Altritol, Mannitol, Glucitol, Gulitol, Iditol, Galactitol, Talitol, Desoxyglucitol, DesoxyMannitol, N-Acetyl-glucitolamin-2, Maltitol und Lactitol, zur Verbesserung der Hemmwirkung einer aus der aus Vanadat (V), oligomerem Vanadat (V) und Gemischen davon bestehenden Gruppe ausgewählten ionischen Verbindung auf ein Enzym mit Phosphatase-Aktivität und der Fähigkeit zur Hydrolyse der Phosphoesterbindung eines Phosphotyrosinrests im Peptid unter SEQ ID NO:1, gelöst in einer gepufferten wässrigen Lösung mit neutralem pH und Ca²⁺-Ionen in einer Konzentration zwischen 0,7 mM und 1,2 mM.

6. Verwendung nach Anspruch 5, **dadurch gekennzeichnet, dass** die Aktivität der Phosphatase auf einen Wert zwischen 1,5% und 40% verringert wird.

7. In-vitro-Verfahren zur Verbesserung der Hemmwirkung einer aus der aus Vanadat (V), oligomerem Vanadat (V) und Gemischen davon bestehenden Gruppe ausgewählten ionischen Verbindung auf ein Enzym mit Phosphatase-Aktivität und der Fähigkeit zur Hydrolyse der Phosphoesterbindung eines Phosphotyrosinrests im Peptid unter SEQ ID NO:1, gelöst in einer gepufferten wässrigen Lösung mit neutralem pH und Ca²⁺-Ionen in einer Konzentration zwischen 0,7 mM und 1,2 mM, wobei das Verfahren die folgenden Schritte umfasst:
(a) Lösen einer Zusammensetzung, die (i) die ionische Verbindung und (ii) einen wasserlöslichen Zuckeralkohol mit 4-100 Kohlenstoffatomen, ausgewählt aus der Gruppe bestehend aus Threitol, Eythritol, Ribitol, Arabinitol, Xylitol, Lyxitol, Desoxyribitol, Desoxyarabinitol, Allitol, Altritol, Mannitol, Glucitol, Gulitol, Iditol, Galactitol, Talitol, Desoxyglucitol, Desoxymannitol, N-Acetylglucitolamin-2, Maltitol und Lactitol, umfasst, wobei das Molverhältnis des Zuckeralkohols und der ionischen Verbindung gleich oder höher als 1:1 ist, in einem wässrigen Lösungsmittel und
(b) Inkontaktbringen des Enzyms mit Phosphatase-Aktivität mit der Lösung aus Schritt (a).

8. Verfahren nach Anspruch 7, **dadurch gekennzeichnet, dass** die Konzentration des Zuckeralkohols in der Lösung zwischen 1 mM und 100 mM liegt.

9. Verfahren nach einem der Ansprüche 7 und 8, **dadurch gekennzeichnet, dass** die Konzentration von Orthovanadat in der Lösung zwischen 0,1 mM und 10 mM liegt.

10. Verfahren nach einem der Ansprüche 7 bis 9, **dadurch gekennzeichnet, dass** die ionische Verbindung und der Zuckeralkohol als Trockensubstanz zu einer das Enzym mit Phosphatase-Aktivität enthaltenden wässrigen Probe gegeben werden.

11. Kit-of-parts, umfassend Verpackungsmaterial und eine Zusammensetzung, die (i) eine aus der aus Vanadat (V), oligomerem Vanadat (V) und Gemischen davon bestehenden Gruppe ausgewählte ionische Verbindung, (ii) einen wasserlöslichen Zuckeralkohol mit 4-100 Kohlenstoffatomen umfasst, wobei das Molverhältnis des Zuckeralkohols und der ionischen Verbindung gleich oder höher als 1:1 ist.

12. Kit nach Anspruch 11, **dadurch gekennzeichnet, dass** die Zusammensetzung zusätzlich ein Reduktionsmittel und/oder einen Chelatbildner für zweiwertige oder dreiwertige positiv geladene Metallionen umfasst.

13. Kit nach einem der Ansprüche 11 und 12, **dadurch gekennzeichnet, dass** die Zusammensetzung als Trockensubstanz in Form einer Tablette bereitgestellt wird.

## Revendications

1. Composition liquide comprenant un solvant aqueux, une enzyme possédant une activité de phosphatase, de l'orthovanadate, du mannitol et du dithiothréitol, **caractérisée en ce que** la concentration du mannitol se situe entre 1 mM et 100 mM et **en ce que** le rapport molaire du mannitol à l'orthovanadate est égal ou supérieur à 1:1.

2. Composition selon la revendication 1, **caractérisée en ce que** la concentration de l'orthovanadate se situe entre 0,1 mM et 10 mM.

3. Composition selon la revendication 2, **caractérisée en ce que** la concentration de l'orthovanadate se situe entre 0,5 mM et 5 mM.

4. Composition selon l'une quelconque des revendications 1 à 3, **caractérisée en ce que** la composition comprend en outre un agent de chélation pour des ions métalliques divalents ou trivalents à charge positive.

5. Utilisation in vitro d'un alcool de sucre soluble dans l'eau comprenant de 4 à 100 atomes de carbone, choisi parmi le groupe constitué par le thréitol, l'érythritol, le ribitol, l'arabitol, le xylitol, le lyxitol, le désoxyribitol, le désoxyarabitol, l'allitol, l'altritol, le mannitol, le glucitol, le gulitol, l'iditol, la galacticol, le talitol, le désoxyglucitol, le désoxymannitol, la N-acétyl-glucitol-amine-2, le maltitol et le lactitol pour augmenter l'effet inhibiteur d'un composé ionique choisi parmi le groupe constitué par le vanadate (V), le vanadate oligomère (V) et leurs mélanges sur une enzyme possédant une activité de phosphatase capable d'hydrolyser la liaison phosphoester d'un résidu de phosphotyrosine dans le peptide de la SEQ ID NO:1 à l'état dissous dans une solution aqueuse tamponnée possédant un pH neutre et contenant des ions Ca²⁺ à une concentration entre 0,7 mM et 1,2 mM.

6. Utilisation selon la revendication 5, **caractérisée en ce que** l'activité de la phosphatase est réduite à une valeur entre 1,5 % et 40 %.

7. Procédé in vitro pour augmenter l'effet inhibiteur d'un composé ionique choisi parmi le groupe constitué par le vanadate (V), le vanadate oligomère (V) et leurs mélanges sur une enzyme possédant une activité de phosphatase capable d'hydrolyser la liaison phosphoester d'un résidu de phosphotyrosine dans le peptide de la SEQ ID NO:1 à l'état dissous dans une solution aqueuse tamponnée possédant un pH neutre et contenant des ions Ca²⁺ à une concentration entre 0,7 mM et 1,2 mM, comprenant les étapes dans lesquelles :
(a) on dissout, dans un solvant aqueux, une composition comprenant (i) le composé ionique et (ii) un alcool de sucre soluble dans l'eau comprenant de 4 à 100 atomes de carbone, choisi parmi le groupe constitué par le thréitol, l'érythritol, le ribitol, l'arabitol, le xylitol, le lyxitol, le désoxyribitol, le désoxyarabitol, l'allitol, l'altritol, le mannitol, le glucitol, le gulitol, l'iditol, la galacticol, le talitol, le désoxyglucitol, le désoxymannitol, la N-acétyl-glucitol-amine-2, le maltitol et le lactitol, le rapport molaire de l'alcool de sucre au composé ionique étant égal ou supérieur à 1:1 ;
(b) on met l'enzyme possédant une activité de phosphatase en contact avec la solution de l'étape (a).

8. Procédé selon la revendication 7, **caractérisé en ce que** la concentration de l'alcool de sucre dans la solution se situe entre 1 mM et 100 mM.

9. Procédé selon l'une quelconque des revendications 7 et 8, **caractérisé en ce que** la concentration de l'orthovanadate dans la solution se situe entre 0,1 mM et 10 mM.

10. Procédé selon l'une quelconque des revendications 7 à 9, **caractérisé en ce qu'**on ajoute le composé ionique et l'alcool de sucre sous la forme d'une matière sèche à un échantillon aqueux contenant l'enzyme possédant une activité de phosphatase.

11. Collection d'éléments juxtaposés comprenant une matière de conditionnement et une composition comprenant (i) un composé ionique choisi parmi le groupe constitué par le vanadate (V), le vanadate oligomère (V) et leurs mélanges, (ii) un alcool de sucre soluble dans l'eau comprenant de 4 à 100 atomes de carbone, le rapport molaire de l'alcool de sucre au composé ionique étant égal ou supérieur à 1:1.

12. Collection selon la revendication 11, **caractérisée en ce que** la composition comprend en outre un agent de réduction et/ou un agent de chélation pour des ions métalliques divalents ou trivalents à charge positive.

13. Collection selon l'une quelconque des revendications 11 et 12, **caractérisée en ce que** la composition est fournie en tant que matière sèche sous la forme d'un comprimé.


### SEQUENCE LISTING

<110> Roche Diagnostics GmbH
   F. Hoffmann-La Roche AG
<120> Enhancement of vanadium-containing phosphatase inhibitor
<130> 24107
<150> EP 07001593.8
   <151> 2007-01-25
<160> 1
<170> PatentIn version 3.2
<210> 1
   <211> 14
   <212> PRT
   <213> Artificial
<220>
   <223> protein phosphatase substrate
<220>
   <221> MISC_FEATURE
   <222> (10)..(10)
   <223> in the substrate peptide the Tyrosine residue is phosphorylated
<400> 1
   Arg Arg Leu Ile Glu Asp Ala Glu Pro Tyr Ala Ala Arg Gly
   1               5                   10
<110> Roche Diagnostics GmbH
   F. Hoffmann-La Roche AG
<120> Enhancement of vanadium-containing phosphatase inhibitor
<130> 24107
<150> EP 07001593.8
   <151> 2007-01-25
<160> 1
<170> PatentIn version 3.2
<210> 1
   <211> 14
   <212> PRT
   <213> Artificial
<220>
   <223> protein phosphatase substrate
<220>
   <221> MISC_FEATURE
   <222> (10)..(10)
   <223> in the substrate peptide the Tyrosine residue is phosphorylated
<400> 1
   Arg Arg Leu Ile Glu Asp Ala Glu Pro Tyr Ala Ala Arg Gly
   1               5                   10